Europäisches Patentamt

European Patent Office

Office européen des brevets

(19)

(11) Publication number: **0 194 158**
A2

# (12) EUROPEAN PATENT APPLICATION

(21) Application number: **86301643.2**

(22) Date of filing: **07.03.86**

(51) Int. Cl.⁴: **C 07 K 15/00**, C 12 P 21/00, C 12 N 15/00, C 12 N 5/00, G 01 N 33/577, G 01 N 33/543, C 07 K 17/08

(30) Priority: **08.03.85 GB 8506102**
**25.02.86 US 831751**

(43) Date of publication of application: **10.09.86**
**Bulletin 86/37**

(84) Designated Contracting States: **AT BE CH DE FR GB IT LI LU NL SE**

(71) Applicant: **Baylor College of Medicine, Texas Medical Center 1200 Moursund Avenue, Houston Texas 77030 (US)**

(72) Inventor: **Langone, John J., 7735 Candlegreen, Houston Texas 77071 (US)**
Inventor: **Van Vunakis, Helen, 60 Thornton Row, Waltham Massachusetts 02154 (US)**
Inventor: **Gjika, Hilda B., 146 Tremont Street, Brighton Massachusetts 02135 (US)**
Inventor: **Bjercke, Robert J., 11927 Stanwood, Houston Texas 77031 (US)**

(74) Representative: **Wilkinson, Stephen John et al, c/o Stevens, Hewlett & Perkins 5 Quality Court Chancery Lane, London WC2A 1HZ (GB)**

(54) Anti-nicotine and anti-cotinine antibodies (monoclonal and other), and their uses in nicotine and cotinine assays.

(57) Disclosed are monoclonal antibodies which bind nicotine, cotinine and/or derivatives thereof, methods for producing these monoclonal antibodies and antibody producing hybridomas for these monoclonal antibodies, and nicotine or cotinine non-isotopic immunoassays using these monoclonal antibodies.

EP 0 194 158 A2

ACTORUM AG

## ANTI-NICOTINE AND ANTI-COTININE ANTIBODIES (MONOCLONAL AND OTHER), AND THEIR USES IN NICOTINE AND COTININE ASSAYS

### Field of the Invention

The invention relates to antibodies (and to monoclonal antibodies), which bind nicotine, cotinine, and/or derivatives thereof; it further includes the use of these products in nicotine or cotinine assays.

### Background of the Invention

Nicotine is the principal alkaloid in tobacco, and in mammals cotinine is its major metabolite. See E.R. Bowman, L.B. Turnbull, H. McKennis, Jr., 127 J. Pharmacol. Exp. Ther. 92 (1959); J. Gorrod, P. Jenner, 6 Essays Toxicol. 35 (1975). Monitoring of cotinine levels is preferred over doing so for nicotine. Cotinine is thought a more reliable indicator of tobacco consumption inasmuch as it has a longer plasma half life, its levels remain relatively constant in active smokers over long periods, and its concentrations are generally significantly higher than those of nicotine, which is rapidly metabolized. See J.J. Langone, H.B. Gjika, H. Van Vunakis, 12 Biochemistry 5025 (1973); J.J Langone, H. Van Vunakis, 84 Methods in Enzymology 628 (1982); N.J. Haley, C.M. Axelrod, T.A. Milton, 73 Amer J. Pub. Health 1204 (1983); N.L. Benowitz, National Institute on Drug Abuse Research Monograph Series No. 48, Measurement in the Analysis and Treatment of Smoking

Behavior p. 6 (1983). (N.L. Benowitz id. also discusses the relative merits of using other products, i.e., carbon monoxide and thiocyanate, as indicia of tobacco consumption.)

Radioimmunoassays (RIAs) 1/ have been used to measure nicotine or cotinine in physiological fluids (J.J. Langone et al., (1973) supra, J.J. Langone, H. Van Vunakis, Radioimmunoassay of Drugs and Hormones in Cardiovascular Medicine, p. 55 (1979)), as has gas chromatography, gas chromatography-mass spectrometry, and high performance liquid chromatography. (See N.L. Benowitz, supra for a comparison of the advantages and limitations of these techniques.) While RIAs are generally satisfactory in studies of active tobacco users, more sensitive assays are needed for "passive smokers" (those involuntarily exposed to tobacco smoke), and so that concentration of large fluid sample volumes is not needed to determine levels during the post-distributional pharmokinetic phase which follows tobacco consumption. Avoidance of the need for concentrating fluid samples is of special importance when monitoring saliva or urine, because variations in their pH or ionic strengths can perturb antigen-antibody binding and cause its inhibition.

---

1/ In radioimmunoassays the antigen is treated to become a radioactively labeled tracer. The amount of antigen-antibody complex formed, which is related directly to the amount of antigen in the test sample under consideration, is determined by measuring the intensity of radioactivity of the complex. For a sample procedure, see Langone et al., Biochemistry (1973) supra.

0194158

Another disadvantage of RIAs is the inherent hazards associated with radioactive materials. It is clear, therefore, that a test is needed which does not use radioactive materials, which is sensitive at low concentrations of antigen, and which has other necessary attributes such as reproducibility (constancy of results), and cost-effectiveness.

To date, the problem with developing a competitive immuno-assay 2/ has been that neither nicotine nor cotinine could be bound to a solid phase such that their functional moieties remained available for antibody binding. This prevented formation of an antibody-antigen complex bound to the solid phase, that bound complex being needed to perform a competitive immuno-assay.

A further problem has been the inability to develop a monoclonal antibody 3/ specific for nicotine, cotinine or their

---

2/ A competitive immuno-assay operates, as its terms imply, by competition among two sets of antigen to bind one set of antibody. In the solid phase competitive assay described below, antigen (derivatives of cotinine or nicotine) are bound to a solid surface and antibody together with a "free" antigen sample is added. The more "free" antigen present, the greater the extent to which it "out-competes" the bound antigen for available antibody. Thus lower levels of antigen bound to the solid-phase correlates with higher levels of "free" antigen in the sample.

3/ An animal has many different kinds of lymphocytes (i.e., special blood or blood plasma cells) each capable of producing an antibody of a particular structure which can seek out a single epitope on an antigen. Once the animal is invaded by an antigen, a lymphocyte which produces an antibody specific for that antigen will reproduce (or clone) in vast numbers to secrete a large supply of antibodies to conquer the invader.

(Footnote Cont'd) derivatives. Monoclonal antibodies generally would allow greater assay accuracy because (among other reasons), the amount of antibody specific to the subject antigen can be more accurately monitored than when using a family undifferentiated antibodies as are present in antisera.

## Summary of the Invention

A conjugate of a polypeptide and a nicotine derivative (3'-hydroxymethylnicotine hemisuccinate and poly-L-lysine) and of a polypeptide and a cotinine derivative (4'-carboxycotinine and poly-L-lysine), which both can be bound to a solid phase, have been developed. Additionally, monoclonal antibodies have been developed, one of which binds nicotine or the nicotine conjugate and the other cotinine or the cotinine conjugate. These products allow practice of an accurate, reproducible enzyme-linked immunoassay (ELISA), which is suitable for measuring low concentrations of antigen and eliminates the need for radioactive materials. Nevertheless, another embodiment within the scope of the invention (described below), employs a

---

3/    (Footnote Cont'd From Previous Page) These antibodies are of identical molecular structure, all being made of the same clones; they are thus termed "monoclonal antibodies." However, in a live animal, even though monoclonal antibodies are produced, they are always found together with other antibodies to other antigens. Therefore, the blood or serum of an animal contains only a mixture known as "polyclonal antibodies," i.e., derived from different (poly)-clones.

radioactive tracer rather than an enzyme for use in quantification.

In brief, the ELISA is performed by binding the conjugate to a solid phase, and adding antibody together with free hapten. 4/ A suitable enzyme is associated with the antibody, and the action of the enzyme on a later-added substrate produces objective, measurable results (typically, flourescense or color intensity), which correlate with the amount of bound antibody. By initially using known concentrations of "free" hapten, a calibration curve of hapten concentration v. "objective results" is developed. When test samples with unknown hapten concentrations are used, the "objective results" obtained from their use are used together with the graph to determine the amount of free hapten.

In a preferred embodiment a monoclonal antibody is used with the ELISA. However, murine (mouse) monoclonal antibody 5/ does not bind well to horse radish peroxidase covalently linked to protein A of Staphylococcus aureus (HrP-SpA); (this is an example of a suitable enzyme/protein conjugate, the enzyme HrP acting on 0-phenylenediamine substrate.) Thus, a second antibody is added which does bind

---

4/ A hapten is a small molecule with an affinity for a particular antibody; in this case, cotinine or nicotine.

5/ Mice are often used to make monoclonal antibodies.

HrP-SpA. This second antibody also binds the murine monoclonal antibody, thereby forming a "sandwich." This second "anti-monoclonal-antibody antibody" is rabbit anti-mouse in a preferred embodiment.

In an alternate embodiment a monoclonal antibody is not used, and instead, rabbit antisera (containing undifferentiated antibodies) is added. It directly binds HrP-SpA and thus no second antibody is needed, and, no "sandwich" is formed.

The invention also includes use of enzyme/protein conjugates other than HrP-SpA, including, but not limited to, a Horse radish Peroxidase-streptavidin conjugate which acts on 0-phenylenediamine substrate, and biotinylated alkaline phosphatase linked through streptavidin, which acts on umbelliferyl phosphate substrate.

Many other enzyme/substrate systems will also be apparent to those skilled in the art. In addition, the SpA can be radioactively labeled (and the enzyme/substrate eliminated), and this system used in the "sandwich" immunoassay of the invention.

### Exemplification of the Invention

A. <u>Example of Making Hybridomas and Suitable Monoclonal Antibodies</u>

As noted above, many different antibodies (poly-clones) are produced <u>in</u> <u>vivo</u> (in a live animal), and the problem is with isolating a family of monoclonal antibodies.  A suitable family of murine monoclonal antibodies to nicotine, cotinine and the above-mentioned derivatives was isolated by the following method.

1.    Formation of Immunogenic Conjugates

Insofar as nicotine and cotinine are non-immunogenic (do not cause production of antibodies in vivo), and also lack functional groups suitable for binding to an immunogenic carrier molecule, they must be altered to make them suitable for binding to an immunogenic carrier.  From nicotine and cotinine 3'-hydroxymethylnicotine hemisuccinate and 4'-carboxycotinine were prepared and coupled covalently to free amino groups of keyhole limpet hemocyanin (KLH), as described in detail in Langone et al., Biochemistry (1973) supra, and Langone and Van Vunakis, Methods in Enzymology (1982), supra. Briefly, 25 mg of 1-ethyl-3 (3-dimethylamino-propylcarbodiimide (EDAC; purchased from Bio-Rad Laboratories, Rockville Centre, NY) in 1.0 ml water at pH 6.8, was added dropwise and with stirring to a solution of 50 mg of the nicotine or cotinine derivative and 50 mg KLH (purchased from Sigma Chemical Co., St. Louis, MO) in 1.0 ml water, at pH 6.8.  After 16 hours at ambient temperature, the immunogens were separated from low molecular weight components by chromatography on a column (1.9 x 35 cm) of Sephadex G-50 M (Pharmacia, Piscataway, NJ),

- 7 -

and wet-packed and eluted with 0.15 M sodium chloride- 0.05 M sodium phosphate at pH 7.2. Fractions (1.5 ml) corresponding to the void volume (predetermined using blue dextran 2000 as the marker) were pooled. The incorporation of the nicotine and cotinine derivatives was estimated to be about 6-18 moles/mole KLH, determined by measuring the difference in absorption of light at a wavelength of 260 nm, (the wavelength at which nicotine and cotinine absorb), between KLH alone and the hapten-KLH conjugate. The hapten derivatives described above were used as standards in this test.

2. <u>Immunization of Mice</u>

Injection of the immunogen (the nicotine or cotinine derivative-KLH conjugate) causes antibody production. These conjugates allow formation of antibodies highly specific to nicotine and cotinine because the area of conjugation to the carrier is distal to each compound's functional groups. <u>See</u> Langone et al. (1973) <u>supra</u>; Langone, Van Vunakis (1982) <u>supra</u>.

Groups of 5 female 8-week-old BALB/c mice (Charles River Breeding Laboratories, Wilmongton, MA) were first immunized and then boosted a total of 3 times, 21 days apart, by intraperitoneal and subcutaneous injections of 100 ug of the immunogen, which was emulsified with an equal volume (0.5 ml) of complete Freund's adjuvant (Cappel Laboratories, Cochranville, PA). As measured by the radioimmunoprecipitation technique described in detail in Langone, et al. (1973), supra, all 5 mice in each group produced serum antibodies to nicotine or cotinine. This radioimmunoprecipitation was performed with optimally diluted rabbit anti-mouse whole Immunoglobin (Ig; Cappel Laboratories), to which was added 10,000 counts per minute (cpm) (S)-(-)-[$^3$H]nicotine (2.4 Ci/mmole; New England Nuclear) or (S)-(-)-[$^3$H]cotinine (2.4 Ci/mmole) prepared from a racemic mixture of [$^3$H]nicotine which had been prepared by an enzymatic procedure described in Langone, Van Vunakis, (1982), supra. These radioactive tracers correspond to the naturally occurring isomers.

Thereafter, the mouse in each group with the highest serum level of anti-nicotine or anti-cotinine antibodies was given an additional 50 ug of the immunogen intravenously in saline. Three days later, spleen cells of the mice were harvested for production of hybridomas.

3.  Fusion of Cells

When spleen cells are fused with myelomas (malignant cells), the product cells (hybridomas) are capable of rapid reproduction and thereby enable secretion of large amounts of antibody.

Cell fusions were performed using modifications of the established techniques described in G. Kohler, C. Milstein, 6 Eur. J. Immunol. 511 (1976). Minced immune spleens were passed through a stainless steel screen and diluted to a 2 x10$^7$

splenocytes/ml of serum-free Dulbecco's minimal essential medium (DMEM). Equal volumes (10 ml each) of splenocytes and non-Ig secreting murine myeloma cells (P3x63-Ag.8.653; 5 x $10^5$/ml) were pelleted and suspended in 1 ml of 50% polyethylene glycol 1450 (Bethesda Research Laboratories, Gaithersburg, MD) that was then diluted to a final concentration of 5% polyethylene glycol over 10 minutes with serum-free DMEM. After centrifugation, the cell pellet was resuspended in complete DMEM containing 10% fetal calf serum, 5% of medium NCTC 109, 1% nonessential minimum essential medium amino acids (100x) and 1% sodium pyruvate (100x) to give 5 x $10^5$cells/ml. Cells were plated onto each of the wells of a 96-well microtiter plates (Costar; Cambridge MA) at 5 x $10^5$ cells/well, and incubated in a humidified 7% $CO_2$ atmosphere overnight at 37°C. On each of the next 3 days, 0.1 ml complete DMEM containing HAT (a selection medium containin 0.1 mM hypoxanthine, 4 x$10^{-6}$M aminopterin, and 1.6 x $10^{-5}$M thymidine) was added to each well. Ten to fourteen days after fusion, when hybridomas reached near confluency, supernatants were screened for antibodies to nicotine and cotinine by immunoprecipitation by the technique taught in Langone et al., (1973) and Langone, Van Vunakis, (1982) using (S)-(-)-[$^3$H]nicotine and (S)-(-)-[$^3$H]cotinine (10,000 cpm added).

0194158

In the nicotine fusion, 444 of 480 wells (92.5%) were positive for cell growth and 40 of the 444 positive wells (9.0%) contained hybridomas that produced antibodies to nicotine. In the cotinine fusion, 478/480 wells (99.6%) showed cell growth and 44 of the 478 positive wells (9.2%) contained cells that produced anti-cotinine antibodies. Only those supernatants which after precipitation produced radioactivity at greater than 8 times background level (70 cpm), when using supernatants from myeloma cells as control, were considered to produce antibodies of sufficient affinity (binding strength) to be used to produce the monoclonal antibodies for the assay. 6/

4. Hybridoma growth and cloning

Thirteen of the suitable nicotine binding and 10 of the suitable cotinine binding hybridomas were cloned (i.e., reproduced), 2-4 times by limiting dilution 7/ in 96-well microtiter plates (Costar). Each well contained a mouse thymocyte feeder layer at a concentration of 10$^4$ cells/well (this acts as a growth stimulant), which had been cultured the previous day. See V.T. Oi, L.A. Herzenberg, Selected Methods

---

6/ With the immunoprecipitation test, a higher count means more antigen is being bound in the well from which the subject supernatant was taken. In other words, more hapten-binding antibody is present in those wells.

7/ "Limiting dilution" means supernatant was diluted to the point where the experimenter was satisfied that there was only one hybridoma present in the fluid in each well.

- 11 -

0194158

in Cellular Immunology p. 351 (1980).  Hybridomas showing a high affinity (measured by radioimmunoprecipitation) were expanded in 24-well cluster plates (Costar), and aliquots of

$5 \times 10^6$ cells to be used later were frozen in liquid nitrogen at each stage of expansion.

Of the original samples of 13 and 10, respectively,

$10^7$ cells of 9 nicotine and 4 cotinine binding cloned hybridomas were injected intraperitoneally into BALB/c mice that had been primed with pristane (Aldrich chemical Co.,

Milwaukee, WI) 2 weeks earlier.  See J.W. Goding, 39 J. Immunol. Methods 285 (1980).  Pristane stimulates production of ascites fluid, causing the hybridomas to grow rapidly in vivo and thus produce a lot of antibody.  After 7-10 days, ascites fluid was collected from each mouse, and fluid from those mice which had been injected with hybridomas from the same well was pooled and then stored at -80°C.

B.  Example of Making a Conjugate of Hapten and Polypeptide

In order that the haptens can be bound to a solid phase, nicotine and cotinine derivatives conjugated to a polypeptide (poly-L-lysine) were prepared.  This was done by a new variation of the procedure described in A.P. Gee, J.J.

Langone, 116 Anal. Biochem. 524 (1981), wherein conjugation of methotrexate and 5-methyltetrahydrofolate is described.  In brief, 1.5 mg of 3-'hydroxymethylnicotine hemisuccinate or 4'-carboxycotinine in 0.3 ml of 0.003 M phosphate buffer, pH

0194158

6.35, was added to 1.0 ml of a 5mg/ml solution of poly-L-lysine, mr = 40,000 (Sigma Chemical Co., St. Louis, MO). 1.0 ml of EDAC at a concentration of 5.0 mg/ml of buffer was added dropwise with stirring and the solution allowed to incubate for 16 hours at 4°C.

C.   Properties of the Polypeptide-Hapten Conjugates

The poly-L-lysine conjugates were used without purification, because the low molecular weight unconjugated components do not bind to the plastic plates (i.e. the solid phase). (Data not shown.) The conjugates could be stored for at least 5 months at 4°C in the presence of 0.02% sodium azide or frozen in 0.03 ml aliquots at -80°C without causing significant loss of antibody binding activity.

To determine the amount of hapten bound to polylysine, 1.0 ml of each product was chromatographed on a Sephadex PD-10 column (Pharmacia) and eluted with 0.003 M phosphate-saline buffer, at pH 6.35 to remove low molecular weight components. The concentration of polylysine was determined using the BCA protein assay (Pierce Chemical Co., Rockford, IL) with known amounts of polylysine as standard. Inasmuch as polylysine does not absorb significantly at 260 nm, nicotine and cotinine were estimated by comparing absorbance at 260 nm with values obtained for samples containing known amounts of hapten derivatives. It was estimated that there were 4.3 moles nicotine and 11.6 moles cotinine equivalents per mole polylysine.

- 13 -

D.  Properties of the Monoclonal Antibodies (McAb)

  1.  Isotyping

      Mammalian antibodies fall into several distinct classes.  Those within a given class are said to be the same isotype.  Antibodies are composed of two "light" polypeptide chains and two "heavy" polypeptide chains, which when folded about each other yield a discrete 3-dimensional structure.  Mamalian light chains are always either "kappa" or "lambda."

      To determine the isotype and light chain composition of the monoclonal antibodies, an ELISA was performed using anti-mouse Ig reagents (Litton Industries, Rockville, MD).  0.1 ml nicotine- or cotinine-polylysine conjugate diluted 1:1000 in 0.003 M phosphate (at pH 7.2), was incubated in flat-bottomed wells of 96-well flexible polyvinyl chloride microtiter plates (Dynatech, Alexandria, VA) for 24 hours at 4°C.  The plates were washed with 0.005 M veronal buffered isotonic saline containing 0.01 M EDTA and 0.1% gelatin, pH 7.4 (VBS-EDTA-gel buffer) and the residual binding sites on the plates were blocked by incubation at 37°C for one hour with 0.2 ml VBS-EDTA containing 0.5% gelatin.  After the plates were washed, McAb (0.1 ml ascites diluted in VBS-EDTA-gel) were added and incubated at 37°C for one hour.  The plates were washed and rabbit IgG antibodies specific for the mouse isotypes IgG1, IgG2a, IgG2b, IgG3, IgM, IgA, or mouse kappa or lambda light chains (0.1 ml of dilution specified by the

- 14 -

manufacturer) were added.  In the wells which contained these different isotypes, the appropriate rabbit antibody would bind that isotype and thus attach to the plate.

The plates were incubated at 37°C for one hour, washed, and to label the bound antibody, 0.1 ml HrP-labeled goat anti-rabbit Ig (1:1,000) was added.  After incubation at ambient temperature for one hour, the plates were washed and 0.1 ml of 0.04% O-phenylenediamine substrate (which produces color when acted upon by HrP) in 0.05 M phosphate-0.025 M citrate buffer, at pH 5.0, containing 0.015% hydrogen peroxide was added, and then incubated at ambient temperature for 10-20 minutes.  The color-producing reaction was stopped by addition of 0.05 ml 4N sulphuric acid, and  absorbance at 490 nm (the wavelength appropriate for measuring the color intensity) was measured with a Bio-Tek ELISA reader.

It was found that of the 9 anti-nicotine antibodies under study, three were IgG1k ("k" meaning kappa light chain), four were IgG1-lambda, one was IgG2ak, and one was IgM-lambda. Of the four anti-cotinine antibodies, two were IgG1k, one was IgG2bk, and one was IgA-lambda.

2.   Properties of the Various Isotypes

The properties of those monoclonal antibodies which had been selected for study were then determined.  The binding affinity constant (K; an indication of binding strength), was measured by the method described in R. Muller, 34 J. Immunol.

Methods 345 (1980). The concentration of antibody in the ascites fluid was also measured by a method described in R. Muller, id.

It was also desirable to determine which isotypes, if any, could bind HrP-SpA directly, so as to determine whether a second antibody was needed and if it affected binding. The results are summarized in Table I (see below).

The results in Table I show that with second antibody (which forms a "sandwich" in the ELISA) much less antibody was needed to bind the same amount of HrP-SpA. Therefore, even those isotypes which do not directly bind HrP-SpA (e.g., IgM, IgA and IgG1) are suitable for use in a "sandwich" assay. It is also interesting to note that far less of those isotypes which do bind SpA directly (IgG2a and IgG2b), is needed to bind an equivalent amount of SpA when the second antibody is used. This effect is likely due to the fact that all rabbit IgG binds SpA (See J.W. Goding, 20 J. Immunol. Methods 241 (1978)), and also indicates binding of multiple rabbit antibodies to each mouse antibody.

3. Antibody Selection and Specificity

It was also desirable to measure the specificity of the monoclonal antibodies for the haptens. That is, to what extent will they bind compounds structurally related to the hapten for which they were designed?

- 16 -

The 9 anti-nicotine and 4 anti-cotinine producing hybridomas described previously were screened to find those which produced antibodies having the greatest specificity for S-(-)-nicotine and S-(-)-cotinine 8/ -- the naturally occurring isomers. 9/ The screening assay also selects against antibodies which are directed to the linkage group joining the haptens to KLH, rather than to the haptens themselves. This linkage group (a carbodiimide) is also common to the conjugates of poly-L-lysine which are bound to the solid phase. Thus, for an accurate ELISA, selection of antibodies specific for free hapten is crucial.

The screening assay was performed with conventional radioimmunoassay techniques, using S-(-)-[³H] nicotine and S-(-)-[³H]-cotinine.

---

8/   (S)-(-)-nicotine, (S)-(-)-cotinine and the other compounds used to determine the specificity of anti-nicotine and anti-cotinine were purchased from commercial sources or were synthesized as reported earlier (J.J. Langone et al., Biochemistry (1973) supra; Langone, H. Van Vunakis, Methods in Enzymology (1982) supra; A. Castonguay, H. Van Vunakis, 84 Methods in Enzymology 641 (1982). (R)-(+)-nicotine was prepared by incubation of a racemic mixture of nicotine enantiomers with Pseudomonas putida, an organism that stereospecifically degrades the natural isomer. See M.C. DeTraglia, A. Tometsko, 39 Appl. Env. Microbiol. 1067 (1980). Dr. A.J. Lyons of the U.S. Dept. of Agriculture at Peoria, IL, supplied the bacteria (NRRL B-8061).

9/   Note that synthesis of the hapten derivatives yields mixtures of streoisomeric products. Thus, the monoclonal antibodies produced would be to all these stereoisomers, because these undifferentiated stereoisomers were used to innoculate the mice.

- 17 -

The specificity of two of the antibodies (anti-nicotine 402C10 and anti-cotinine 305E5) was analyzed, using a modification of the sandwich ELISA technique, which technique is described in detail below. In essence, specificity was determined by discerning how much structually related substance need be added to inhibit binding of monoclonal antibody to solid phase hapten by 50%. The assays were carried out in wells coated with 0.012 ug nicotine- or cotinine-polylysine conjugate, and with anti-nicotine and anti-cotinine diluted 1:729,000.

The modification involved use of 0.01 ml of serially diluted standard nicotine or cotinine, (known amounts of inhibitors to assess antibody specificity), or test samples (eg. saliva) added to 0.04 ml casein buffer before its incubation with monoclonal antibodies. Maximum $A_{4,0}$ values (i.e., maximum antibody binding) were determined using casein buffer (0.01 ml) in place of inhibitor. In "Control wells," with no antibody binding, buffer was used in place of McAb. Control values were less than 0.05 absorbance unit. All results are presented below in Table II as they appeared after duplicate or triplicate determinations.

It can be seen from Table II that with antinicotine, 6-hydroxynicotine (not a metabolite) was the most effective of the compounds tested; only 5 times more being required for 50% inhibition than required when using (S-(-)-nicotine. Other nicotine metabolites (e.g. nicotine N'-oxide), cotinine derivatives (e.g. desmethylcotinine and cotinine n-oxide), related tobacco alkaloids (e.g. nornicotine, anabasine, and myosmine), and naturally occurring substances (e.g. nicotinamide) were poor inhibitors. Twenty-five fold more R-(+)-nicotine was required for 50% inhibition as compared with the naturally occuring isomer. Stereospecificity for (S)-(-)-nicotine (5-45 fold) was observed for all 9 anti-nicotine McAb, and this result reflects the selectivity inherent in the above-discussed screening assay.

Similarly, it can be seen in Table II that anticotinine 305E5 is specific for (S)-(-)-cotinine. Even desmethylcotinine (not a metabolite) was a relatively ineffective inhibitor with 13-fold more required for 50% inhibition compared to homologous hapten. Several other compounds that contain only the pyridine, pyrrolidine, or pyrrolidone ring system did not inhibit more than 30% in either immunoassay at the 1,000 ng level (data not shown).

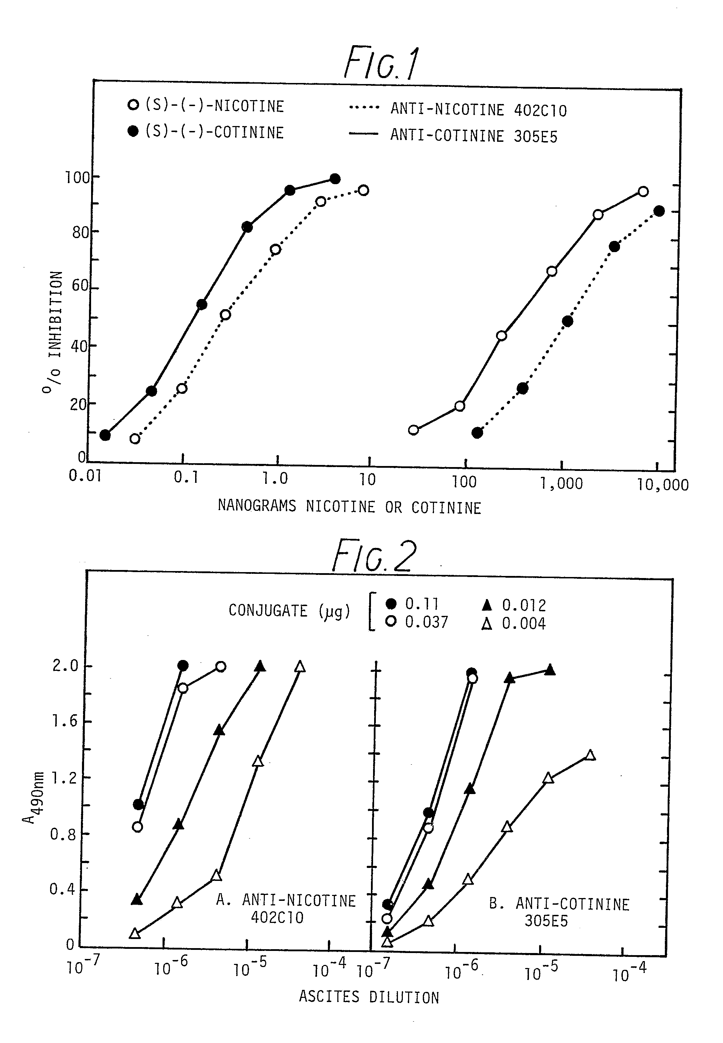

Another measure of assay sensitivity is comparison of the "wrong/right" hapten-monoclonal antibody binding, conducted on (S)-(-)-nicotine and (S)-(-)-cotinine using monoclonal antinicotine 402C10 and anti-cotinine 305E5 with both the haptens, (shown in Fig. 1.)

With antinicotine, 0.25 ng nicotine and 1,000 ng cotinine were required for 50% inhibition. In contrast, as

- 19 -

little as 0.05 ng nicotine gave 15% inhibition. With anticotinine, only 0.12 ng cotinine inhibited binding by 50%, whereas 450 ng of nicotine was required for equivalent inhibition. Only 0.02 ng cotinine inhibited by 15%. Similar inhibition curves (data not shown) using homologous hapten have been obtained for each McAb diluted to give $A_{490}$ values of 1.0–1.2 in the absence of inhibitor. With the 9 antinicotine antibodies, 50% inhibition was obtained with between 0.091–0.51 ng (S)-(-)-nicotine. With the 4 anticotinine antibodies, between 0.038–0.12 ng (S)-(-)-cotinine gave 50% inhibition.

E.  Example of Determination of Binding Conditions and Strength

    1.  Plate Preparation

        The ELISA for nicotine and cotinine was carried out with a new variation of, although in a somewhat similar manner to, the general solid-phase ELISA technique described in A.P. Gee, J.J. Langone, 116 Anal. Biochem. 524 (1981).

        Wells of 96-well polyvinly chloride microtiter plates (Dynatech) were coated by incubation overnight at 4°C with 0.1 ml of the nicotine- or cotinine-poly-L-lysine conjugate diluted with 0.003 M phosphate buffer, pH 6.35.  The wells were then washed once with 0.2 ml casein buffer (0.01 M Tris-buffered saline, Ph 7.6, containing 0.01 M EDTA and 0.2% vitamin-free casein obtained from Nutritional Biochemicals, Cleveland, Ohio), and the free sites on the plates were blocked with the same buffer by incubation at ambient temperature for 1 hour. 10/  At this point, the plates can be stored at 4°C for at least 7 days without affecting the assay.  The plates were then washed once with 0.2 ml casein buffer and the assay was carried out in a total volume of 0.1 ml.

---

    10/  In preliminary experiments it was found that with 0.2% casein in the buffer, the background absorbance at 490 nm in control wells was insignificant (less than 0.05 units) and readings obtained upon replication differed by less than 10%. In contrast, when gelatin, ovalbumin, chicken IgG, or BSA (up to 0.5%) was used in place of casein, the background was significantly higher (the $A_{490}$ was about 0.1-0.3 in wells without monoclonal antibody) and the precision was not as good.

2. <u>Example of Determining Binding</u>

After the preparation, to determine McAb binding, 0.05 ml diluted ascites fluid was added to 0.05 ml casein buffer and the plates were incubated overnight at 4°C. After the wells were washed 3 times with 0.2 ml casien buffer, excess 0.1 ml rabbit IgG anti-mouse IgG (or anti-mouse whole Ig when IgM or IgA monoclonals were used) was added and the plates incubated at ambient temperature for 1.5 hour. The wells were then washed 3 times with casein buffer and incubated with 0.1 ml HrP-SpA (Zymed Laboratories, South San Francisco, CA) at room temperature for 1 hour. After 6 washes with 0.2 ml PBS-0.05% Tween 20, pH 7.4, 0.1 ml of O-phenylenediamine substrate (as used for isotyping) was added and incubated for 20-25 minutes at ambient temperature. Color development was stopped by addition of 0.05 ml 4N sulfuric acid, and the $A_{490}$ nm values were measured in the ELISA reader.

3. <u>Determination of Optimal Conditions</u>

Optimal concentrations for coating of conjugate, and of McAb, were determined from binding curves as shown below in Fig. 2 for the antibodies anti-nicotine 402C10 and anti-cotinine 305E5. Serial dilutions of ascites fluid were added to wells coated by incubation with conjugate equivalent to between 0.004 - 0.11 ug polylysine added. This range corresponds to 0.13-3.4 ng nicotine or 0.25-6.8 ng cotinine added. The "antibody titer", defined as the reciprocal of the ascites dilution required to give an $A_{490}$ value within the practical working range of the colorimeter (e.g. 1.0-1.5) could be established as standard assay conditions. In practice, the combination of reagents that gives optimal assay sensitivity (see below) and is most economical in terms of ascites fluid or conjugate is chosen.

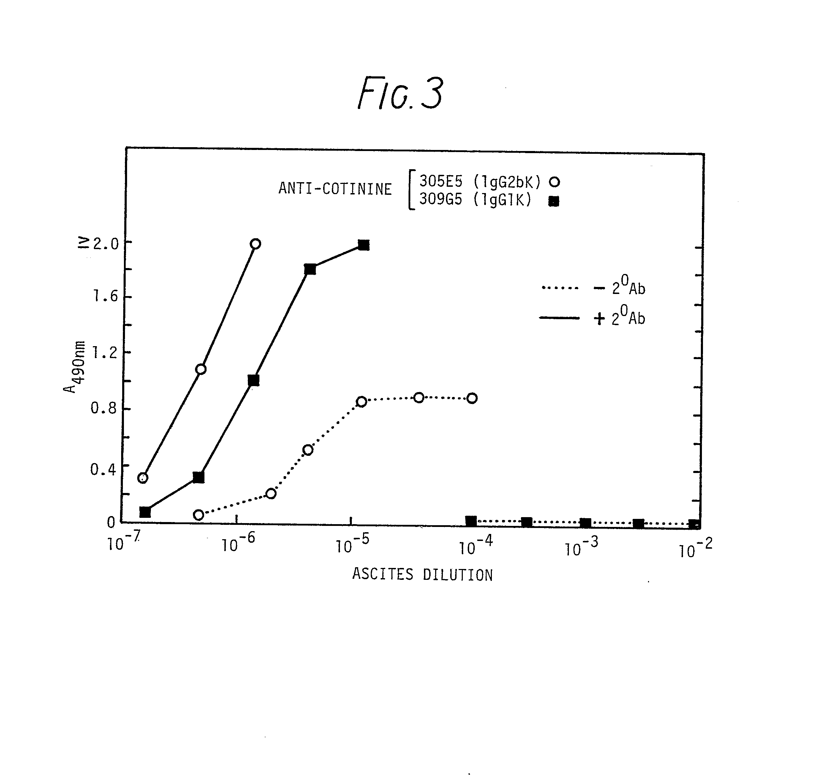

The advantage of using rabbit IgG anti-mouse Ig in a sandwich assay, as opposed to an assay without second antibody, is demonstrated by the representative results shown below in FIG.3, (N.B. in FIG.3, all coated wells were prepared with 0.012 $\mu$g cotinine-polylysine;-2$^O$AB = no second antibody added; + 2$^O$AB = with second antibody added). The anticotinine antibody 309G5 (IgG1k isotype) does not bind HRP-SpA directly, even at a 1:100 dilution. However,the titer was approximately 729,000 in the sandwich assay showing high binding at a low concentration. The other antibody used (305E5) is IgG2bk isotype and binds HRP-SpA in the absence of rabbit anti-mouse IgG. However, $A_{490}$ reached a plateau value of 0.90, even with antibody diluted to 1:9,000. This result suggests that the cotinine conjugate bound to the well, which was present in 0.012 $\mu$g for all runs, 11/ was the limiting reagent. However, when rabbit seond antibody was used,the titer was greater than 2 x 10$^6$ (i.e. binding increased significantly), and $A_{490}$ values greater than 2.0 were obtained even with ascites diluted 1;729,000 or less.

The results summarized in Table I also show that the titer of both anti-nicotine and anti-cotinine was markedly enhanced in the sandwich ELISA.

---

11/ Therefore, a single preparation was sufficient for over 400,000 assays. Also prepared were conjugages using 0.3 and 0.06 mg 4'-carboxycotinine (data not shown),but no advantage was found in terms of assay sensitivity or antibody titer.

F.    Example of an Assay of Saliva Sample

Saliva samples were collected from several non-smokers and analyzed for nicotine and cotinine. Those samples that were negative, i.e. in which nicotine or cotinine could not be detected within the limits of the ELISA (5 ng nicotine and 2 ng cotinine/ml of saliva) were supplemented with different concentrations of nicotine or cotinine and assayed to determine recovery 12/ as well as intra- 13/ and inter-assay 14/ coefficients of variation. It can be seen in Table III (attached in back) that the assays are accurate over a range of concentrations from 5-500 ng/ml. The coefficients of variation are also good, exceeding 10% only for the lowest level of nicotine (10 ng/ml) and cotinine (5 ng/ml).

In addition, assays for nicotine and cotinine were done on saliva samples from 24 subjects, 8 of whom claimed to be non-smokers. Eight non-smokers were correctly identified by both the ELISA and conventional RIA, as were those who claimed to be smokers. Three of those non-smokers had low levels of

---

12/ "Recovery" simply means determining whether the amount of hapten added is ultimately recovered.

13/ "Intra-assay coefficient of variation" is a measure of variations in results between runs performed on the same day.

14/ "Inter-assay coefficient of variation" is a measure of variations in results between runs performed on different days.

alkaloids in saliva (less than 25 ng/ml) which could be detected with the ELISA. This may indicate that their "passive" smoking could be detected by the more sensitive ELISA.

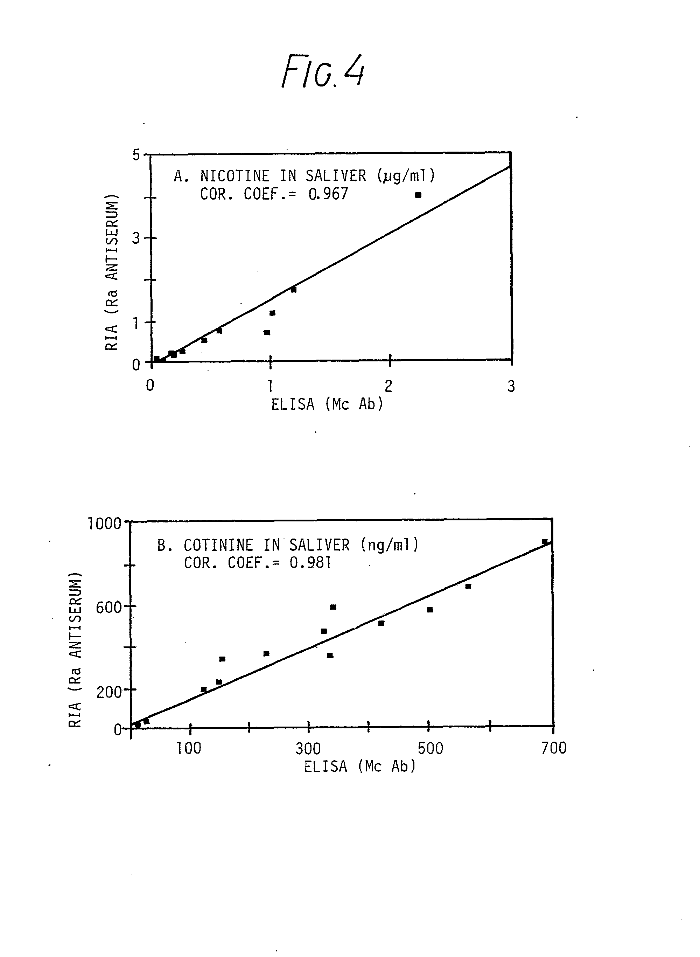

The results shown below in Fig. 4 show good correlation (the correlation coefficient is near to 1.0) between results with the ELISAs and coventional RIAs.

0194158

0194158

Standard curves were also obtained with
(S)-(-)-nicotine or (S)-(-)-cotinine prepared in pooled saliva
from four non-smokers and all dilutions were made in casein
buffer. The cotinine levels tested were 5, 50 and 500 ng/ml,
reflecting the expected concentrations for passive, light, and
heavy smokers. 15/ Even in the presence of undiluted control
saliva (10 ul), there was no interference by non-specific
antibody binding or non-specific inhibition as compared to
standard results obtained using buffer (data not shown).

After testing, these saliva samples were collected for
another study and kept frozen at -20° for 1-2 years. Nicotine
and cotinine are stable under these storage conditions.

G.    Example of an ELISA with Rabbit Antisera

Also developed were similar ELISAs using rabbit
antisera, that being prepared as described in J.J. Langone et
al., Biochemistry (1973) supra; J.J. Langone, H. Van Vunakis,
Methods in Enzymology (1982) supra. In these assays, no second
antibody is required, because all rabbit IgG binds HRP-SpA
(J.W. Goding, 20 J. Immunol. Methods 241 (1978)). With the 6
cotinine antisera, 50% inhibition values ranged between 0.3-1.2
ng cotinine and antibody titers ranged between

_____

15/    Note that Cotinine is not present in tobacco,
however, its concentration in saliva is similar to that in
plasma.

100,000-150,000. With the single rabbit antinicotine serum tested, 3.9 ng nicotine was required for 50% inhibition. The antibody titer was 9,000. Therefore the ELISAs were significantly more sensitive with McAb and the titers of McAb were uniformly higher than when using rabbit antisera. In addition, the inhibition curves were much steeper with McAb, 16/ likely because of the inherent sensitivity of the screening done when using McAb and the presence in the rabbit heterosera of antibodies directed against the conjugate-hapten linkage group (data not shown).

It is also interesting to note that the rabbit antisera could be diluted 40-50 times further in the ELISA and still achieve sensitivity comparable to the conventional RIA.

H.  Examples of Other Uses for the Monoclonal Antibodies

1.  Other Enzyme/Protein Systems

As noted previously, it is possible to use protein/enzyme systems other than HrP-SpA to measure the amount of bound antigen. Proteins other than SpA are needed to perform the ELISA with serum or plasma because IgG present therein reacts with SpA and alters results. While a multitude

---

16/  Note that a steeper inhibition curve (obtained by plotting % inhibition v. amount of antigen present, e.g. Fig. 1), is desirable, for it shows that less antigen is needed to bind an equal amount of available antibody than the antigen needed where the curve is shallow. This enhanced binding yields better intra-assay correlation.

of alternatives will be apparent to those skilled in the art, only a few examples are shown below.

a.   ELISA - Streptavidin - Biotin System:   The wells of polyvinyl chloride microtiter plates (Dynatech Laboratories) were coated respectively with 0.012 and 0.024 ug of polylysine-cotinine and -nicotine, (0.1 ml) in 3 mM phosphate buffer, and incubated overnight at pH 6.3., 4°C.   After aspiration, the wells were blocked with casein buffer for 1 hour at room temperature, washed once with 200 ul of the same buffer, and aspirated again.   Assays were performed in 0.1 ml total volume per well using 0.050 ml diluted ascites, 0.025 ml sample, and 0.025 ml of nicotine or cotinine (whichever is appropriate), for the inhibition curve.   Plates were incubated overnight at 4°C, washed three times with 200 ul of casein buffer, and then 100 ul of biotin conjugated sheep anti-mouse IgG (Cooper Biomedical) diluted 1/500 with casein buffer was added.   After incubation for 4 hours at room temperature, the plates were washed, and then 0.100 ml of horseradish peroxidase-conjugated streptavidin (Zymed) diluted 1/500 with casein buffer was added.   After incubation for 90 minutes at room temperature, the plates were washed five times with 200 ul of PBS containing 0.05% Tween-20, 100 ul of 0-phenylenediamine (10 mg/ml, diluted 1:10 with phosphate-citrate buffer), substrate was added, and the reaction was stopped after 20-30 minutes of incubation at room temperature by addition of 0.050 ml of 4N $H_2SO_4$. Optical density at 490 nm was determined for each well in a EL-310 EIA reader (Bio-Tek Instruments, Burlington, VT).

A number of other possible enzyme/substrate combinations, suitable for use in a colorimetric ELISA, are listed below in Table V.

b.  Fluorometric ELISA:  The wells of polystyrene microtiter plates (Immulon 2; Dynatech Laboratories, Alexandria, VA) were coated respectively with 0.004 and 0.016 ug of polylysine-cotinine and polylysine-nicotine conjugate (0.1 ml) in 3 mM phosphate buffer, and incubated overnight at 4°C, pH 6.3.  After aspiration, the wells were blocked with 0.2 ml casein buffer (0.01M Tris-buffered saline, pH 7.6, containing 0.01M EDTA and 0.2% vitamin-free casein (Nutritional Biochemicals, Cleveland, OH)) for 1 hour at room temperature, and then washed once with 0.2 ml of the same buffer and again aspirated.  Assays were performed in 0.1 ml total volume per well using 0.050 ml diluted ascites (0.025 ml sample), and 0.025 ml nicotine or cotinine.  Plates were incubated overnight at 4°C, washed three times with 0.2 ml casein buffer and then 0.1 ml biotin conjugated sheep anti-mouse IgG (Cooper Biomedical, Malvern, PA) diluted 1/250 with casein buffer was added.  After incubation for 3 hours at room temperature, the plates were washed three times with 0.2 ml casein buffer, and streptavidin (0.5 ug) was added to each well, incubated for 1 hour at room temperature and washed as before. 0.1 ml of biotinylated alkaline phosphatase (Zymed, South San Francisco, CA) diluted 1/200 was added to each well, the plates were incubated for 1 hour at room temperature, washed with 0.2 ml PBS containing 0.05% Tween-20, and then washed three times with 0.2 ml of 0.01 M Tris-buffered saline, pH 7.6. 0.1 ml of substrate was added containing 2.5 mM 4-methylumbelliferyl phosphate dissolved in buffer containing diethanolamine (20% v/v) pH 10, 1 mM $MgCl_2$, and 9 mM levamisol. Fluorescence was measured with a Microfluor fluorometer (Dynatech Laboratories) at an excitation wavelength of 365 nm and emission wavelength of 450 nm. The plates were read right after substrate was added and again at 30 minute intervals. (Plates should be stored in the dark).

The results obtained for various antibodies are shown in Table IV (see below).

Another enzyme/substrate combination (and other detection methods as well), suitable for use with a fluorescence ELISA are listed below in Table VI.

## 2. Radioactive Labelling

It is also possible to use radioactive quantification with the monoclonal antibody assay. For example, [$^{125}$I]SpA can be used either in the microtiter plate assay, or with larger volumes (e.g. final volume of 1.0 ml) in plastic tubes coated with polylysine conjugates. However, [$^{125}$I]SpA
afforded no apparent advantage over the microtiter plate ELISA
in terms of sensitivity or convenience, and although larger
volumes of test sample (e.g. 0.1 ml) could be assayed, more
reagents were required. It also proved less sensitive for
detection of nicotine and cotinine by a factor of ten.

3. Monoclonal Antibodies in a Conventional RIA

It seems it would also be possible to use McAb to
improve a conventional RIA (Langone et al., (1973) supra;
Langone, Van Vunakis (1982) supra), inasmuch as the
[$^{125}$I]-labelled hapten has a linkage group similar to the
hapten-polylysine and hapten-immunogen conjugates. The
screening step with the McAb improves results because when
using undifferentiated poly-clonal antibodies in an RIA, some
are attracted to the linkage group thus reducing the assay
sensitivity.

4. Monoclonal Antibodies in Further Studies

In addition to using the ELISAs (or the similar
procedures) for large scale studies of the health risk for
active and passive tobacco consumers, the McAb described above
could themselves be used as immunogens to generate
anti-idiotypes specific for the anti-nicotine antibody
combining site. See N.K. Jerne, 125C Ann. Immunol. (Inst.
Pasteur) 373 (1974); C.A. Bona, H. Kohler, Monoclonal and

- 33 -
32

Anti-Idiotypic Antibodies:  Probes for Receptor Structure and Function p. 141 (1984).  These anti-idiotypes could be useful in the study of brain receptors specific for (S)-(-)-nicotine.

## Deposit

The following organisms are available from and have been deposited at Baylor College of Medicine, 1200 Moursund Avenue, Houston, Texas 77030.

Anti-nicotine 301C4

Anti-nicotine 302A12

Anti-nicotine 402C10

Anti-nicotine 302G3

Anti-nicotine 303C5

Anti-nicotine 303C10

Anti-nicotine 203G3

Anti-nicotine 304A4

Anti-nicotine 304A7

Anti-cotinine 305E5

Anti-cotinine 206F8

Anti-cotinine 308C11

Anti-cotinine 309G5

These deposits are available to the public from Baylor College of Medicine, the assignee. It should be understood, however, that the availability of a deposit does not constitute a license to practice the subject invention in derogation of patent rights granted by governmental action.

I. Final Observations

It should be emphasized that the above descriptions of the monoclonal antibodies, the polypeptide conjugates, the hybridomas, and the test procedures are all exemplary only and not intended as limiting. In that regard, it is noted that other monoclonal antibodies that bind nicotine and cotinine, and other hybridomas which produce such antibodies and tracer molecules which can be used in such immunoassays, are within the scope of the invention, as are all other procedures using these or related products. The protection for the invention is limited only by the scope of the claims which follow, and further includes all equivalents of the subject matter of those claims.

TABLE I

PROPERTIES OF MONOCLONAL ANTIBODIES

| Monoclonal Antibody | Isotype | $K$ $(M^{-1} \times 10^{-8})$ | Antibody Concentration in Ascites Fluid (mg/ml) | Antibody Titer ($\times 10^{-3}$)* | |
| --- | --- | --- | --- | --- | --- |
| | | | | Without second Antibody** | With second Antibody** |
| Anti-nicotine | | | | | |
| 301C4 | IgG$_1$lambda | 1.03 | 5.79 | 8 | 5,200 |
| 302A12 | IgG$_1$k | 1.25 | 3.54 | 3 | 2,000 |
| 402C10 | IgG$_{2a}$k | 1.21 | 2.36 | 200 | 1,000 |
| 302G3 | IgG$_1$lambda | 1.62 | 4.25 | 3 | 4,200 |
| 303C5 | IgG$_1$lambda | 1.40 | 2.23 | 0.1 | 350 |
| 303C10 | IgG$_2$k | 0.54 | 7.40 | 1 | 2,187 |
| 203G3 | IgMlambda | 0.48 | 7.46 | less than 0.1 | 14 |
| 304A4 | IgG$_1$k | 1.33 | 4.71 | less than 0.1 | 5,000 |
| 304A7 | IgG$_1$lambda | 0.88 | 1.72 | 3 | 560 |
| Anti-cotinine | | | | | |
| 305E5 | IgG$_{2b}$k | 5.68 | 0.59 | more than 9 | 2,187 |
| 206F8 | IgAlambda | 1.85 | 1.09 | less than 0.1 | 60 |
| 308C11 | IgG$_1$k | 3.12 | 0.60 | less than 0.1 | 100 |
| 309G5 | IgG$_1$k | 7.55 | 0.68 | less than 0.1 | 729 |

*/ "Antibody titer" is defined as 1/dilution of ascites ($\times 10^{-3}$) required to give $A_{450}$ nm = 1.0, under standard assay conditions using wells coated with 0.012 ug nicotine or cotinine-polylysine conjugate.

**/ "Second antibody" is the IgG fraction of rabbit anti-mouse IgG, except for anti-nicotine 203G3 and anti-cotinine 206F8, with which anti-mouse whole Ig was used.

TABLE II

SPECIFICITY OF ANTI-NICOTINE AND ANTI-COTININE MONOCLONAL ANTIBODIES*

| Compound* | Nanograms required for 50% inhibition* | |
| --- | --- | --- |
| | Anti-nicotine 402C10 | Anti-cotinine 305E5 |
| (S)-(-)-nicotine | 0.25 | 450 |
| (S)-(-)-cotinine | 1,000 | 0.12 |
| (R)-(+)-nicotine | 6.4 | 320 |
| 6-hydroxynicotine | 1.3 | 330 |
| nicotine N'-oxide | 81.0 | 490 |
| (-)-nornicotine | 95.0 | 50.0 |
| N'-nitrosonornicotine | more than 700 (31%) | 6.5 |
| N-ethylnornicotine | 5.4 | more than 1,000 (44%) |
| desmethylcotinine | 10,000 | 1.6 |
| (-)-cotinine N'-oxide | more than 1,000 (0%) | 4.6 |
| gamma-(3-pyridyl-gamma-oxobutyric acid | 1,920 | 410 |
| anabasine | 14.3 | 170 |
| myosmine | more than 700 (13%) | 220 |
| 2-aminonicotine | 14.0 | 1,700 |
| 6-aminonicotine | 35.0 | 8,500 |
| 1-methyl-2-pyrrolidine ethanol | 5.4 | 6,950 |

*Several pyridine, pyrrolidine and pyrrolidone derivatives (see J.J. Langone, H. Van Vunakis (1982), supra), that were tested gave 0-30% inhibition at the 1,000 ng level.

TABLE III

RECOVERY OF NICOTINE AND COTININE ADDED TO SALIVA

|  | Amount Of Nicotine Added (ng/ml) | | |
|---|---|---|---|
|  | 500 | 50 | 10 |
| Recovery (mean +SD): | | | |
| Intra-assay (n=12) | 489 ± 19 | 51.3 ± 3.5 | 8.3 ± 1.1 |
| Coefficient of variation | 3.9% | 6.8% | 13.3% |
| Inter-assay (n=7) | 486 ± 41 | 51.2 ± 3.8 | 12.5 ± 2.0 |
| Coefficient of variation | 8.4% | 7.4% | 16.4% |

|  | Amount Of Cotinine Added (ng/ml) | | |
|---|---|---|---|
|  | 500 | 50 | 10 |
| Recovery (mean +SD): | | | |
| Intra-assay (n=12) | 504 ± 19 | 47.7 ± 1.2 | 5.3 ± 0.50 |
| Coefficient of variation | 3.8% | 2.5% | 9.4% |
| Inter-assay (n=7) | 510 ± 13 | 47.9 ± 3.3 | 5.0 ± 0.68 |
| Coefficient of variation | 2.5% | 6.9% | 13.6% |

TABLE IV

Properties of Mouse Monoclonaland Rabbit Anti-Cotinine

Antibodies As Measured Using The Fluorescent ELISA

| Monoclonal Antibody | Antibody Titer $(x\ 10^{-3})$ | I 50% |
|---|---|---|
| 305E5 | 2450 | 0.024 |
| 206F8 | 70 | 0.255 |
| 308C11 | 200 | 0.033 |
| 309G5 | 670 | 0.015 |
| Rabbit Antibody | | |
| 113D | 1030 | 0.045 |
| 481D | 700 | 0.215 |
| 497D | 700 | 0.048 |
| 495D | 370 | 0.310 |
| 494D | 1030 | 0.075 |
| 497D | 370 | 0.100 |
| 482D | 430 | 0.025 |

"Antibody Titer $(X10^{-3})$" is defined as 1/dilution of monoclonal antibody ascites or rabbit anti-sera to give one-half maximum fluorescence units.

"I 50%" is defined as the nanograms of cotinine inhibitor required for 50% inhibition.

TABLE V

Colorimetric ELISA

| Enzyme-label* | Examples of Suitable Substrates |
|---|---|
| Horseradish peroxidase | 0-tolidine |
| | 2-2 azino-di-(3 ethylbenzothiazolin) sulfone-6 (ABTS) |
| | 0-dianisidine |
| | 5-amino-salicylic acid |
| | diamino benzidine |
| | 3-methyl-2-benzothiozolinone hydrozone and 3-(dimethylamino) benzoic acid |
| | 3-amino-9-ethylcarbazole |
| Beta-galactosidase | Nitrophenyl galactoside |
| | p-nitrophe yl-Beta-D-galactopyranoside |
| Alkaline phosphatase | p-nitrophenyl phosphate |
| Glucose oxidase | B-D-glucose |
| | glucose-p-nitrobluetetrazolium chloride |
| | glucose-p-phenazine methosulfate |
| Urease | urea |
| | pH indicator - bromocresol purple |

*Enzyme or fluorochrome may be conjugated to anti-nicotine or anti-cotinine antibody or to second antiglobulin antibody, or to protein A, streptavidin, or biotin.

TABLE VI

Fluorescence ELISA

| Enzyme label* | Examples of Suitable Substrates and/or Detection Systems |
|---|---|
| B-galactosidase | 4-methylumbelliferyl-Beta-D-galactoside |

Fluorichrome-label*

| fluorescein (FITC) | Detected with a fluorescent microscope |
|---|---|
| rhodamine (RITC) | or fluorometer |
| Texas Red | |

---

*Enzyme or fluorochrome may be conjugated to anti-nicotine or anti-cotinine antibody or to second antiglobulin antibody, or to protein A, streptavidin, or biotin.

0194158

<u>What Is Claimed Is</u>:

1. A monoclonal antibody which binds to nicotine or a conjugate comprising 3'-hydroxymethylnicotine hemisuccinate and poly-L-lysine.

2. The monoclonal antibody of claim 1 which is a murine antibody.

3. The monoclonal antibody of claim 1 which is IgM, IgA, IgG1, IgG2a or IgG2b.

4. A monoclonal antibody which binds to cotinine or a conjugate comprising 4'-carboxycotinine and poly-L-lysine.

5. The monoclonal antibody of claim 4 which is a murine antibody.

6. The monoclonal antibody of 4 which is IgM, IgA, IgG1, IgG2a or IgG2b.

7. A process of making a monoclonal antibody which binds nicotine or cotinine comprising:

   making a hybridoma which produces a nicotine or cotinine binding monoclonal antibody;

   isolating the nicotine or cotinine binding monoclonal antibody.

8. The process of claim 7 wherein the hybridoma is a murine hybridoma made by :

   coupling a derivative of nicotine or cotinine to a protein carrier;

   immunizing a mouse with the protein-hapten conjugate; and

   fusing splenocytes from the mouse with non-immumoglogubin secreting murine myeloma cells.

9. The process of claim 8 wherein the derivative is 3'-hydroxymethylnicotine hemisuccinate or 4'-carboxycotinine and the protein derivative is keyhole limpet hemocyamin, further including the steps of:

   cloning the resulting hybridomas;

   growing the cloned hybridomas intraperitoneally prior to isolating the nicotine or cotinine binding monoclonal antibody.

- 35 -
41

0194158

10. A monoclonal antibody formed by the process of any of claims 7, 8 or 9.

11. A hybridoma which secretes a monoclonal antibody which binds nicotine or a conjugate comprising 3'-hydroxymethylnicotine hemisuccinate and poly-L-lysine.

12. The hybridoma of claim 11 which is a murine hybridoma.

13. The hybridoma of claim 11 formed by fusing a myeloma cell with an immunogenic cell which produces anti-nicotine antibodies.

14. The hybridoma of claim 13 wherein the immunogenic cell is formed by coupling nicotine to an immunogenic carrier molecule and immunizing a subject animal with the resultant conjugate.

15. The hybridoma of claim 14 wherein the carrier molecule is keyhole limpet hemocyanin and the subject animal is a mouse.

16. A hybridoma which secretes a monoclonal antibody which binds cotinine or a conjugate comprising 4'-carboxycotinine and poly-L-lysine.

17. The hybridoma of claim 16 which is a murine hybridoma.

18. The hybridoma of claim 16 formed by fusing a myeloma cell with an immunogenic cell which produces anti-cotinine antibodies.

19. The hybridoma of claim 18 wherein the immunogenic cell is formed by coupling cotinine to an immunogenic carrier molecule and immunizing a subject animal with the resultant conjugate.

20. The hybridoma of claim 19 wherein the carrier molecule is keyhole limpet hemocyanin and the subject animal is a mouse.

21. An assay for determining the presence of nicotine or cotinine in animal fluids comprising adding to the animal fluid a pharmacologically effective amount of a monoclonal antibody which binds nicotine or a conjugate comprising 3'-hydroxymethylnicotine hemisuccinate and poly-L-lysine.

22. The method of claim 21 wherein the assay is non-isotopic.

23. The method of claim 22 wherein the assay is an enzyme-linked immunosorbent assay.

- 36 -
42

24. The method of claim 23 wherein the enzyme is horse radish peroxidase or biotinylated alkaline phosphatase and it respectively acts on 0-phenylenediamine substrate or umbelliferyl phosphate substrate.

25. The method of any of claims 21 to 24 wherein the antibody is a murine monoclonal antibody.

26. The method of any of claims 21 to 24 wherein the animal body fluid assayed is human saliva or urine.

27. The method of claim 25 wherein the animal body fluid assayed is human saliva or urine.

28. The method of any of claims 21 to 24 wherein the assay is a sandwich-type solid phase assay, in which a conjugate comprising 3'-hydroxymethylnicotine hemisuccinate and poly-L-lysine is bound to the solid phase and an anti-antibody binds the monoclonal antibody.

29. The method of claim 25 wherein the assay is a sandwich-type solid-phase assay, in which a conjugate comprising 3'-hydroxymethylnicotine hemisuccinate and poly-L-lysine is bound to the solid phase and rabbit anti-mouse antibody binds the murine monoclonal antibody.

30. A method of using a monoclonal antibody which binds nicotine in an animal body fluid sandwich-type solid phase enzyme linked immunosorbent assay for nicotine, comprising:

binding the enzyme to an anti-monoclonal-antibody antibody;

binding a conjugate comprising 3'-hydroxymethylnicotine hemisuccinate and poly-L-lysine to the solid phase;

adding free nicotine and monoclonal antibody.

31. The method of claim 30 wherein the enzyme is horse radish peroxidase or biotinylated alkaline phosphatase, the monoclonal antibody is a murine antibody, and the anti-monoclonal-antibody antibody is rabbit anti-mouse.

32. A method of using a monoclonal antibody which binds cotinine or a conjugate comprising 4'-carboxycotinine and poly-L-lysine in an animal body fluid assay for cotinine.

33. The method of claim 32 wherein the assay is non-isotopic.

34. The method of claim 33 wherein the assay is an enzyme-linked immunosorbent assay.

35. The method of claim 34 wherein the enzyme is horse radish peroxidase.

36. The method of any of claims 32 to 35 wherein the antibody is a murine monoclonal antibody.

37. The method of any of claims 32 to 35 wherein the animal body fluid assayed is human saliva or urine.

38. The method of claim 36 wherein the animal body fluid assayed is human saliva or urine.

39. The method of any of claims 32 to 35 wherein the assay is a sandwich-type solid phase assay, in which a conjugate comprising 4'-carboxycotinine and poly-L-lysine is bound to the solid phase and an anti-antibody binds the monoclonal antibody.

40. The method of claim 36 wherein the assay is a sandwich-type solid phase assay, in which a conjugate comprising 4'-carboxycotinine and poly-L-lysine is bound to the solid phase and rabbit anti-mouse antibody binds the murine monoclonal antibody.

41. A method of using a monoclonal antibody which binds cotinine in an animal body fluid sandwich-type solid phase enzyme linked immunosorbent assay for cotinine, comprising:

    binding the enzyme to an anti-monoclonal-antibody antibody;

    binding a conjugate comprising 4'-carboxycotinine and poly-L-lysine to the solid phase;

    adding free cotinine and monoclonal antibody.

42. The method of claim 41 wherein the enzyme is horse radish peroxidase or biotinylated alkaline phosphatase, the monoclonal antibody is a murine antibody, and the anti-monoclonal-antibody antibody is rabbit anti-mouse.

43. A complex comprising a monoclonal antibody bound to nicotine or a conjugate comprising 3'-hydroxymethylnicotine hemisuccinate and poly-L-lysine.

44. The complex of claim 43 wherein the monoclonal antibody is a murine monoclonal antibody.

45. The complex of claim 43 wherein the monoclonal antibody is IgM, IgA, IgG1, IgG2a or IgG2b.

46. The complex of any of claims 43 to 45 further including an anti-monoclonal-antibody antibody bound thereto.

47. The complex of claim 46 further including an enzyme linked to the anti-monoclonal-antibody antibody.

48. The complex of claim 46 wherein the anti-monoclonal-antibody antibody is rabbit anti-mouse.

49. A complex comprising a monoclonal antibody which is bound to cotinine or a conjugate comprising 4'-carboxycotinine and poly-L-lysine.

50. The complex of claim 49 wherein the monoclonal antibody is a murine monoclonal antibody.

51. The complex of claim 49 wherein the monoclonal antibody is IgM, IgA, IgG1, IgG2a or IgG2b.

52. The complex of any of claims 49 to 51 further including an anti-monoclonal-antibody antibody bound thereto.

53. The complex of claim 52 further including an enzyme linked to the anti-monoclonal-antibody antibody.

54. The complex of claim 52 wherein the anti-monoclonal-antibody antibody is rabbit anti-mouse.

55. A conjugate comprising 3'hydroxymethylnicotine hemisuccinate and poly-L-lysine.

56. The conjugate of claim 55 bound to plastic.

57. The conjugate of claim 55 or 56 having an antibody bound thereto.

58. The conjugate of claim 57 having an enzyme associated therewith.

59. The conjugate of claim 57 wherein the antibody is a monoclonal antibody.

60. A conjugate comprising 4'-carboxycotinine and poly-L-lysine.

61. The conjugate of claim 60 bound to plastic.

62. The conjugate of claim 60 or 61 having an antibody bound thereto.

63. The conjugate of claim 62 having an enzyme associated therewith.

64. The conjugate of claim 62 wherein the antibody is a monoclonal antibody.

65. A method of using a conjugate of 3'-hydroxymethylnicotine hemisuccinate and poly-L-lysine in a solid phase competitive assay, comprising the steps of:

linking the conjugate to a solid phase;

adding free nicotine and antibody at the same time;

quantifying the amount of bound antibody.

66. The method of claim 65 wherein the antibody is a monoclonal antibody.

67. The method of claim 65 wherein the antibody is among those found in rabbit antisera, and the antibody addition step is performed by adding rabbit antisera.

68. The method of claim 65 or 67 wherein an enzyme associated with the antibody and acting on a substrate is used to determine the amount of bound antibody.

69. A method of using a conjugate of 4'-carboxycotinine hemisuccinate and poly-L-lysine in a solid phase competitive assay, comprising the steps of:

linking the conjugate to a solid phase;

adding free nicotine and antibody at the same time;

quantifying the amount of bound antibody.

70. The method of claim 69 wherein the antibody is a monoclonal antibody.

71. The method of claim 69 wherein the antibody is among those found in rabbit antisera, and the antibody addition step is done by adding rabbit antisera.

72. The method of claim 69 or 71 wherein an enzyme associated with the antibody and acting on a substrate is used to determine the amount of bound antibody.

73. A conjugate comprising a polypeptide linked to a hapten such that upon binding of the polypeptide to a solid phase, the hapten functional groups remain available for antibody binding.

74. The conjugate of claim 73 wherein the polypeptide is poly-L-lysine and the hapten is 4'-carboxycotinine or 3'-hydroxymethylnicotine hemisuccinate.

1/3

0194158

FIG.1

FIG.2

# FIG.3

# FIG.4

A. NICOTINE IN SALIVER (μg/ml)
COR. COEF.= 0.967

B. COTININE IN SALIVER (ng/ml)
COR. COEF.= 0.981